# EUROPEAN PATENT SPECIFICATION

(11) **EP 0 301 799 B1**
(45) Date of publication and mention of the grant of the patent: **24.11.1993**
(21) Application number: 88306854.6
(22) Date of filing: 26.07.1988
(51) Int. Cl.: C07C 243/24, C07C 311/49, G03C 1/06

(54) **Photographic high contrast silver halide elements and process using them**
Photographisches Silberhalogenid-Element mit hohem Kontrast und Verfahren zu seiner Verwendung
Elément photographique d'halogénure d'argent à haut contraste et procédé de son utilisation

(30) Priority: 28.07.1987 GB 8717782
(43) Date of publication of application: 01.02.1989
(73) Proprietor: EASTMAN KODAK COMPANY (a New Jersey corporation), Rochester, New York 14650 (US); KODAK LIMITED, Hemel Hempstead Herts, HP1 1JU (GB)
(72) Inventor: Coldrick, Philip John, Pinner Middlesex HA5 2LR (GB); Sidhu, Jasbir, North Wembley Middlesex, HA0 3JB (GB)
(74) Representative: Baron, Paul Alexander Clifford

(56) References cited:
- EP-A- 0 196 626
- DE-A- 3 041 923
- US-A- 4 278 748

## Description

This invention relates to photographic high contrast silver halide elements and to methods of obtaining high contrast photographic images therewith.

For many years the very high contrast photographic images needed in the graphic arts and printing industries were obtained by developing a 'lith' emulsion (usually high in silver chloride content) in a hydroquinone, low sulphite, 'lith' developer by the process known as infectious development. However, such low sulphite developers are inherently unstable and are particularly inappropriate for machine processing.

Recently, emulsions containing hydrazide nucleating agents have been used and processed in a developer with conventional amounts of sulphite, hydroquinone and possibly metol or a pyrazolidone.

Many hydrazides have been proposed for incorporation in such high contrast silver halide materials, for example in US Patents 4,323,643, 4,278,748, 4,031,127 and 4,030,925. In particular, US Patent 4,323,643 describes aryl hydrazides of the formula: 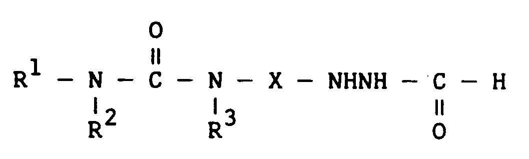
wherein
R¹ and R² each represents hydrogen, an aliphatic group, an aromatic group, or a heterocyclic group;
R³ represents hydrogen or an aliphatic group, and
X represents a divalent aromatic group.

European Patent Application 196,626 also descrives photographic materials useful for obtaining high contrast negative images using various hydrazide nucleating agents. These agents include sulfonamidophenyl hydrazides as well as alkyl substituted phenoxybutyramidohydrazide compounds.

It is known that when hydrazides from different classes are used in combination, a superior result can often be obtained. Some known commercial products contain such a combination.

The present invention provides novel hydrazide nucleators effective in high contrast photographic material some of which when used alone provide as good a photographic material as was previously obtainable only with a combination of hydrazides.

According to the present invention there is provided an aryl hydrazide of the formula: 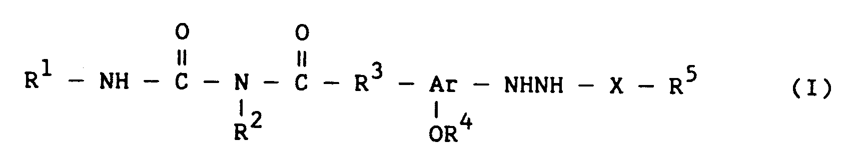
where
X is SO₂ or CO,
R¹ and R² (which may be the same or different) each represents an aliphatic group of at least 2 carbon atoms, a cycloaliphatic group, a substituted or unsubstituted aromatic group or a substituted or unsubstituted heterocyclic group;
R³ represents -(CH₂-)ₙ- where n is an integer of 1 to 4, preferably 1 or 2,
R⁴ represents a substituted or unsubstituted aliphatic or a substituted or unsubstituted aromatic group,
R⁵ represents hydrogen or an aliphatic, aromatic or heterocyclic group any of which groups being substituted or unsubstituted, provided that when X is SO₂, R⁵ is not hydrogen,
Ar represents an aromatic group which may contain alkyl or alkoxy groups in addition to R³ and OR⁴.

Preferably the group - OR⁴ is ortho or para to the hydrazide group. The group R³ is preferably meta to the hydrazide group.

These hydrazides may be incorporated into silver halide negative-working materials to provide very high contrast images of high image quality on development at a pH between 10.5 and 12.0 with little or no pepper fog.

The present invention also provides a photographic element comprising a support and a layer of a photosensitive silver halide emulsion containing in or adjacent said layer an aryl hydrazide of formula (I) as defined above.

Examples of groups which R¹ and R² may represent are alkyl groups of 2-12 carbon atoms, e.g. ethyl, propyl, butyl, n-pentyl, t-pentyl, n-octyl and n-dodecyl; cycloalkyl groups, e.g. cyclohexyl; aryl groups, e.g. phenyl, naphthyl nitrophenyl, tolyl; and heterocyclic groups, e.g. pyridine or piperidine.

Examples of groups which R³ may represent are methylene and ethylene.

Examples of groups which R⁴ may represent are an alkyl group of 1-12 carbon atoms, preferably 1-6 carbon atoms, for example methyl, ethyl, propyl, butyl, hexyl, dodecyl, or benzyl or aryl groups, for example, phenyl.

Examples of groups which R⁵ may represent are hydrogen or alkyl groups having up to 7, preferably up to 5, carbon atoms and their halo-, alkoxy- or phenyl- substituted derivatives; cycloalkyl groups, e.g. cyclohexyl, methylcyclohexyl, bornyl or isobornyl; aromatic groups, e.g. phenyl or substituted phenyl e.g. p-methyl-phenyl; or a heterocyclic group, e.g. pyridyl or piperidinyl.

The aryl group Ar may, for example be a phenyl or napthyl group which may contain further alkyl or alkoxy substituents.

A preferred embodiment of the present invention comprises hydrazides of the general formula: 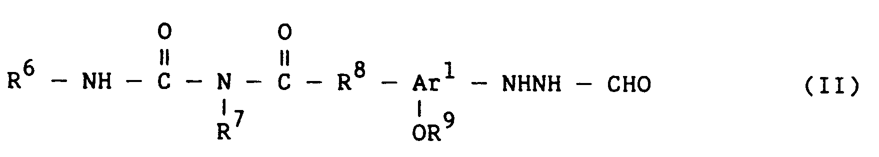
where
R⁶ and R⁷ (which may be the same or different) each represents an aliphatic group of at least 2 carbon atoms or a cycloaliphatic group,
R⁸ represents -(CH₂-)ₙ- where n is an integer of 1 to 4, preferably 1 or 2,
R⁹ represents a substituted or unsubstituted aliphatic group,
Ar represents an aromatic group.

Such hydrazides when used alone provide as good a result as previously obtained by a combination of hydrazides.

The hydrazides of the present invention may be prepared by reacting an aryl hydrazide of the formula: 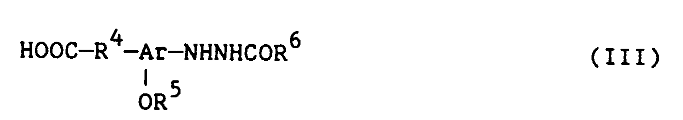
with a carbodiimide of the formula:

R¹-N = C = N - R² (IV)

The hydrazide nucleating agents described herein are preferably present in the photographic elements and emulsions of this invention in a concentration of from 10⁻⁴ to 10⁻¹ mol per mol of silver. A preferred quantity of the hydrazide compound is from 5 x 10⁻⁴ to 5 x 10⁻² mol per mol of silver. Optimum results are obtained when the hydrzide compound is present in a concentration of from 8 x 10⁻⁴ to r x 10⁻³ mol per mol of silver. The hydrazide compound can be incorporated in a photographic silver halide emulsion layer or, alternatively, the hydrazide compound can be present in a hydrophilic colloid layer of the photographic element. Preferably, such hydrophilic colloid layer is coated contiguous to the emulsion layer in which the effects of the hydrzide compound are desired. The hydrazide compound can also be present in the photographic element in other layers such as subbing layers, interlayers or overcoating layers.

The compounds of formula (III) may be prepared by known methods. For example, p-aminophenylacetic acid may be diazotised and the resulting diazo compound reduced with, for example, stannous chloride to form the corresponding hydrazine.

Typically the reaction may be accomplished by stirring III and IV in a solvent, e.g. dimethylformamide, at room temperature.

The silver halide emulsion may be any of the negative-working emulsions described in Research Disclosure, December 1978, Item 17643, Sections I and II published by Industrial Opportunities Ltd., The Old Harbourmaster's, 8 North Street, Emsworth, Hants P010 7DD, U.K. This publication will be identified hereafter as "Research Disclosure". The grains of the emulsions may be of any size and shape, for example the grains may be cubic, octahedral or tabular. Tabular grain emulsions are described, for example, in British specifications 2,109,576, 2,112,157 and 2,110,830. Suitable vehicles for the emulsion layers and other layers of elements of this invention are described in Research Disclosure Section IX and the publications cited therein.

The photographic elements of this invention or individual layers thereof, can contain brighteners (see Research Disclosure Section V), antifoggants and stabilizers (see Research Disclosure Section VI), antistain agents and image dye stabilizer (see Research Disclosure Section VII, paragraphs I and J), light absorbing and scattering materials (see Research Disclosure Section VIII), hardeners (see Research Disclosure Section XI), plasticizers and lubricants (see Research Disclosure Section XII), antistatic agents (see Research Disclosure Section XIII), matting agents (see Research Disclosure Section XVI) and development modifiers (see Research Disclosure Section XXI).

The photographic elements can be coated on a variety of supports as described in Research Disclosure Section XVII and the references described therein.

The following examples illustrate the preparation and use of hydrazides according to the present invention.

### Example 1

### Preparation of 1-formyl-2-{3-[2-(1,3-cyclohexylureido)carbonyl]ethyl-4-methoxyphenyl} hydrazine

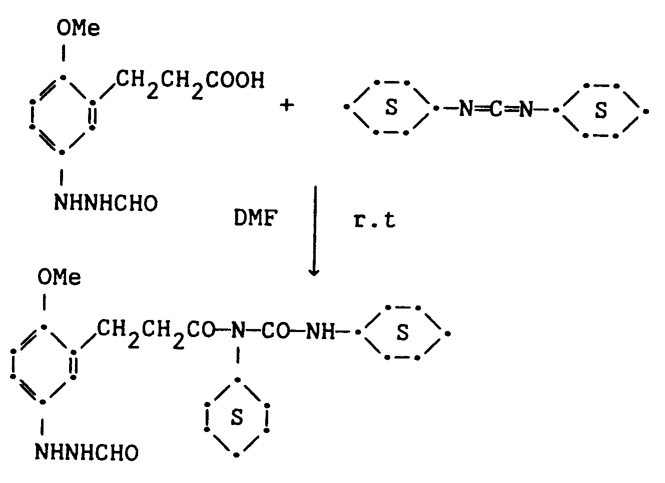

3-[5-(2-formylhydrazino)-2-methoxyphenyl] propionic acid (2.38g, 0.01m) and dicyclohexyl carbodimide (4.12, 0.02m) in dry dimethylformamide (25ml) was stirred at room temperature for 12 hours. At the end of the reaction time the reaction mixture was filtered and the filtrate was poured onto ice/H₂O. The solid was filtered and purified by column chromatography to afford 63% of the required product.

### Example 2

### (a) Control Coating

A cubic bromoiodide emulsion (2.5 mole % iodide; mean grain size 0.23 µm) was coated on a polyester support at 3.5 g/m²Ag and 2.6 g/m² gelatin, and contained the spectral sensitizing dye anhydro-5,5'-dichloro-9-ethyl-3,3'-bis (3-sulphopropyl) oxocarbocyanine hydroxide, triethylamine salt at 216mg/Ag mole, the nucleating agents 1-formyl-2-{4[2,4-di-tert-pentylphenoxy -butyramido]phenyl} hydrazine (formula A below) at 373mg/Ag mole and 1-formyl-2-[4-(hexylureido)phenyl] hydrazine (formula B below) at 72mg/Ag mole; and the addenda oleic ether of polyethylene glycol-1540 at 250 mg/Ag mole, 4-hydroxy-6-methyl-1,3,3a,7-tetraazaindene sodium salt at 1g/Ag mole, a latex copolymer of methyl acrylate, 2-acrylamido-2-methylpropanesulphonic acid, sodium salt and 2-acetoacetoxy ethyl methacrylate (88:5:7 by weight) at 34g/Ag mole, 4-carboxymethyl-4-thiazoline-2-thione at 53mg/Ag mole, hydroquinone at 4.7g/Ag mole, and 4,5-dihydroxy-1,3-benzene disulphonic acid, disodium salt at 3.2g/Ag mole. The emulsion was overcoated with 1.3g/m² gelatin. The coating was hardened with bis(vinylsulphonylmethyl) ether at 4.9% of the total weight of gelatin.

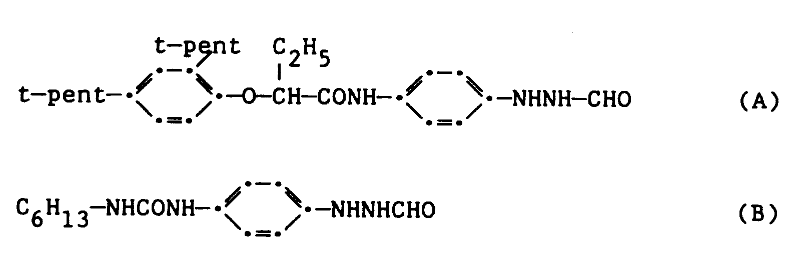

### (b) Invention

Prepared as described above (2a) except that the two nucleators were replaced with 400mg/Ag mole of 1-formyl-2-{3-[2-(1,3-dicyclohexylureido)carbonyl] ethyl-4-methoxyphenyl]hydrazine prepared in Example 1.

The coatings were exposed through a 0.1 logE density step tablet for 10s to the light from a 6V 48W tungsten bulb suitably attenuated. Sensitometric data were determined after tray development for 80s at 30° C in fresh developer having the formula:

| | Grams/l |
|---|---|
| Sodium bromide | 3.0 |
| Orthophosphoric acid, 75% solution | 47.4 |
| Sodium metabisulfite | 52.5 |
| Sodium hydroxide 50% | 50.0 |
| 5-Methylbenzotriazole | 0.25 |
| Hydroquinone | 35.0 |
| 4-Hydroxymethyl-4-methyl-phenyl-3-pyrazolidinone | 0.30 |
| 3-diethylamino-1,2-propanediol | 19.7 |
| Water to | 1.0 liter |
| Adjust with 50% Sodium hydroxide solution to pH 11.4 | |

This data is shown in Table 1. Upper scale contrast is the gradient of a straight line drawn between the density points 2.5 and 4.0 above fog on the sensitometric curve.

**Table 1**

| Coating | Dmax | Dmin | Relative Speed | Upper Scale Contrast |
|---|---|---|---|---|
| 2a | 5.30 | 0.02 | 100 | 9.8 |
| 2b | 5.62 | 0.02 | 98 | 11.14 |

The results show that there is very little sensitometric difference between the two coatings. Image quality differences were also very small.

## Claims

1. An aryl hydrazide having the formula: 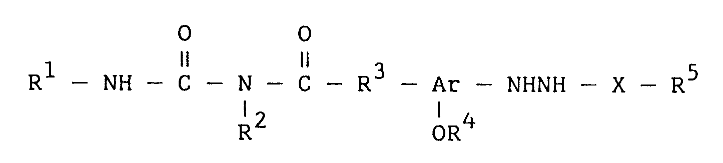 where
X is SO₂ or CO,
R¹ and R² (which may be the same or different) each represents an alkyl group having 2-12 carbon atoms, a cycloalkyl group, a phenyl, naphthyl, nitrophenyl, tolyl, pyridine or piperidine group;
R³ represents -(CH₂-)ₙ- where n is an integer of 1 to 4, preferably 1 or 2,
R⁴ represents an alkyl group having 1-12 carbon atoms or a benzyl or phenyl group,
R⁵ represents hydrogen or an alkyl group having up to seven carbon atoms, or a halo, alkoxy, or phenyl derivative, or a cycloalkyl, phenyl, methylphenyl pyridyl or piperidinyl group, provided that when X is SO₂, R⁵ is not hydrogen,
Ar represents a phenyl or napthyl group which may contain alkyl or alkoxy groups in addition to R³ and OR⁴.

2. An aryl hydrazide as claimed in Claim 1 in which the group -OR⁴ is ortho or para to the hydrazide group.

3. An aryl hydrazide as claimed in Claim 1 or 2 in which the group -R³ is meta to the hydrazide group.

4. An aryl hydrazide as claimed in Claim 1 having the general formula: 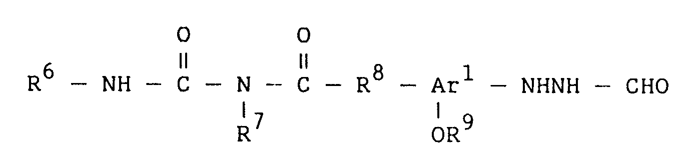 where
R⁶ and R⁷ (which may be the same or different) each represents an alkyl group having 2-12 carbon atoms or a cycloalkyl group,
R⁸ represents -(CH₂-)ₙ- where n is an integer of 1 to 4, preferably 1 or 2,
R⁹ represents an alkyl group having 1-12 carbon atoms,
Ar represents a phenyl or naphthyl group.

5. The aryl hydrazide 1-formyl-2-{3-[2-(1,3-cyclohexylureido)carbonyl]ethyl-4-methoxyphenyl} hydrazine

6. A photographic element comprising a support and a layer of a photosensitive silver halide emulsion containing as nucleating agent in or adjacent said layer an aryl hydrazide of formula 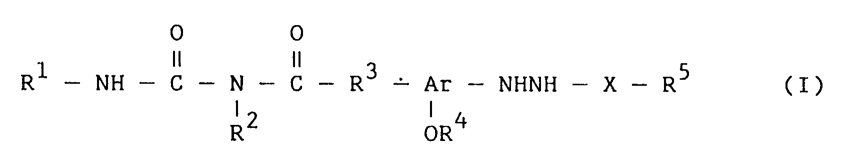 where
X is SO₂ or CO,
R¹ and R² (which may be the same or different) each represents an aliphatic group of at least 2 carbon atoms, a cycloaliphatic group, a substituted or unsubstituted aromatic group or a substituted or unsubstituted heterocyclic group;
R³ represents -(CH₂-)ₙ- where n is an integer of 1 to 4, preferably 1 or 2,
R⁴ represents a substituted or unsubstituted aliphatic or a substituted or unsubstituted aromatic group,
R⁵ represents hydrogen or an aliphatic, aromatic or heterocyclic group any of which groups being substituted or unsubstituted, provided that when X is SO₂, R⁵ is not hydrogen,
Ar represents an aromatic group which may contain alkyl or alkoxy groups in addition to R³ and OR⁴.

7. A photographic element as claimed in Claim 6 which is a negative-working high contrast material.

8. A method of forming photographic high contrast images which comprises imagewise exposing a photographic element according to Claims 6 or 7 and developing at a pH of from 10.5 to 12.0.

## Patentansprüche

1. Ein Arylhydrazid der Formel: 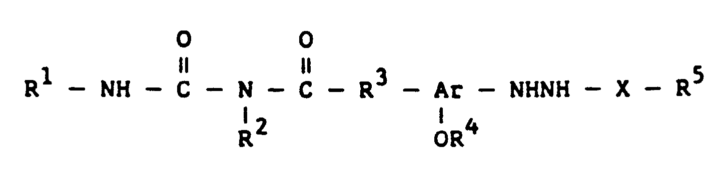 worin bedeuten:
X gleich -SO₂ oder -CO-,
R¹ und R² (die gleich oder verschieden sein können) jeweils eine Alkylgruppe mit 2 bis 12 Kohlenstoffatomen, eine Cycloalkylgruppe, eine Phenyl-, Naphthyl-, Nitrophenyl-, Tolyl-, Pyridin- oder Piperidingruppe;
R³ gleich -(CH₂-)ₙ-, worin n für eine ganze Zahl von 1 bis 4, vorzugsweise 1 oder 2 steht,
R⁴ eine Alkylgruppe mit 1 bis 12 Kohlenstoffatomen oder eine Benzyl- oder Phenylgruppe,
R⁵ ein Wasserstoffatom oder eine Alkylgruppe mit bis zu 7 Kohlenstoffatomen oder ein Halo-, Alkoxy- oder Phenylderivat, oder eine Cycloalkyl-, Phenyl-, Methylphenyl-, Pyridyl- oder Piperidinylgruppe, wobei gilt, daß wenn X für -SO₂- steht, R⁵ kein Wasserstoffatom ist,
Ar eine Phenyl- oder Naphthylgruppe, die Alkyl- oder Alkoxygruppen zusätzlich zu R³ und OR⁴ aufweisen kann.

2. Ein Arylhydrazid nach Anspruch 1, worin die Gruppe -OR⁴ in ortho- oder para-Position zur Hydrazidgruppe angeordnet ist.

3. Ein Arylhydrazid nach Anspruch 1 oder 2, worin die Gruppe -R³ sich in meta-Position zur Hydrazidgruppe befindet.

4. Ein Arylhydrazid nach Anspruch 1 mit der allgemeinen Formel: 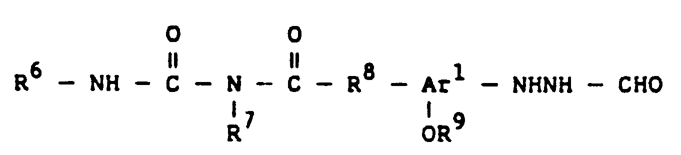 worin bedeuten
R⁶ und R⁷ (die gleich oder verschieden sein können) jeweils eine Alkylgruppe mi 2 bis 12 Kohlenstoffatomen oder eine Cycloalkylgruppe,
R⁸ die Gruppe -(CH₂-)ₙ-, worin n für eine ganze Zahl von 1 bis 4, vorzugsweise 1 oder 2 steht,
R⁹ eine Alkylgruppe mit 1 bis 12 Kohlenstoffatomen,
Ar eine Phenyl- oder Naphthylgruppe.

5. Das Arylhydrazid 1-Formyl-2-{3-[2-(1,3-cyclohexylureido)carbonyl]ethyl-4-methoxyphenyl}hydrazin.

6. Photographisches Element mit einem Träger und einer Schicht aus einer photosensitiven Silberhalogenidemulsion, die als keimbildendes Mittel in oder benachbart zu der Schicht ein Arylhydrazid der Formel 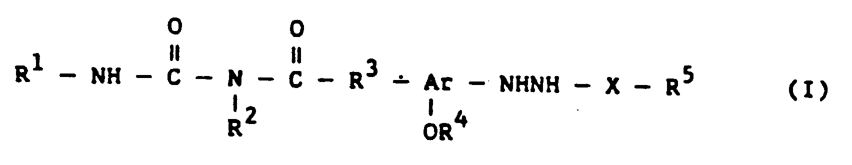 enthält, worin bedeuten:
X gleich -SO₂- oder -CO-,
R¹ und R² (die gleich oder verschieden sein können) jeweils eine aliphatische Gruppe mit mindestens 2 Kohlenstoffatomen, eine cycloaliphatische Gruppe, eine substituierte oder unsubstituierte aromatische Gruppe oder eine substituierte oder unsubstituierte heterocyclische Gruppe;
R³ eine Gruppe der Formel -(CH₂-)ₙ-, worin n eine ganze Zahl von 1 bis 4, vorzugsweise 1 oder 2 ist,
R⁴ eine substituierte oder unsubstituierte aliphatische oder eine substituierte oder unsubstituierte aromatische Gruppe,
R⁵ Wasserstoff oder eine aliphatische, aromatische oder heterocyclische Gruppe, wobei eine jede dieser Gruppen substituiert oder unsubstituiert sein kann, wobei gilt, daß wenn X für -SO₂- steht, R⁵ nicht die Bedeutung eines Wasserstoffatomes hat,
Ar eine aromatische Gruppe, die Alkyl- oder Alkoxygruppen zusätzlich zu R³ und OR⁴ aufweisen kann.

7. Photographisches Element nach Anspruch 6, bestehend aus einem negativ arbeitenden hoch kontrastreichen Material.

8. Verfahren zur Herstellung von hoch kontrastreichen photographischen Bildern, bei dem man ein photographisches Element nach Anspruch 6 oder 7 exponiert und bei einem pH-Wert von 10,5 bis 12,0 entwickelt.

## Revendications

1. Aryl hydrazide de formule : 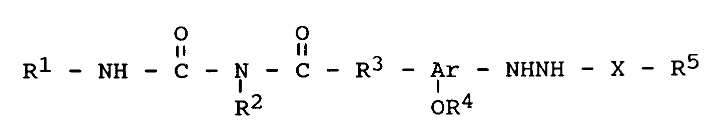 où
X est SO₂ ou CO,
R¹ et R² (qui peuvent être les mêmes ou différents) représentent chacun un groupe alkyle ayant de 2 à 12 atomes de carbone, un groupe cycloalkyle, phényle, naphtyle, nitrophényle, tolyle, pyridine ou pipéridine ;
R³ représente -(CH₂)ₙ- où n est un entier de 1 à 4, de préférence 1 ou 2,
R⁴ représente un groupe alkyle ayant de 1 à 12 atomes de carbone ou un groupe benzyle ou phényle,
R⁵ représente l'hydrogène ou un groupe alkyle ayant jusqu'à 7 atomes de carbone, ou un dérivé halo, alkoxy ou phényle, ou un groupe cycloalkyle, phényle, méthylphényle, pyridyle ou pipéridinyle, avec la condition que lorsque X est SO₂, R⁵ ne soit pas un hydrogène,
Ar représente un groupe phényle ou naphtyle qui peut contenir des groupes alkyle ou alkoxy en plus de R³ et OR⁴.

2. Aryl hydrazide selon la revendication 1 dans lequel le groupe -OR⁴ est en position ortho ou para du groupe hydrazide.

3. Aryl hydrazide selon la revendication 1 ou 2 dans lequel le groupe -R³ est en position méta par rapport au groupe hydrazide.

4. Aryl hydrazide selon la revendication 1 de formule : 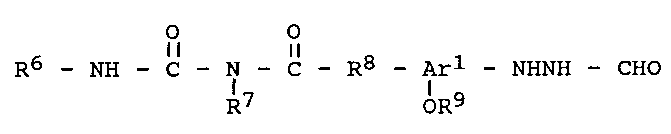 où
R⁶ et R⁷ (qui peuvent être les mêmes ou différents) représentent chacun un groupe alkyle de 2 à 12 atomes de carbone ou un groupe cycloalkyle,
R⁸ représente -(CH₂)ₙ- où n est un entier de 1 à 4, de préférence 1 ou 2,
R⁹ représente un groupe alkyle de 1 à 12 atomes de carbone,
Ar représente un groupe phényle ou naphtyle.

5. Aryl hydrazide 1-formyl-2{3-[2-(1,3-cyclo- hexyluréido) carbonyl]éthyl-4-méthoxyphényl}hydrazine.

6. Produit photographique comprenant un support et une couche d'émulsion aux halogénures d'argent photosensible contenant comme agent de nucléation dans cette couche ou dans une couche adjacente un arylhydrazide de formule : 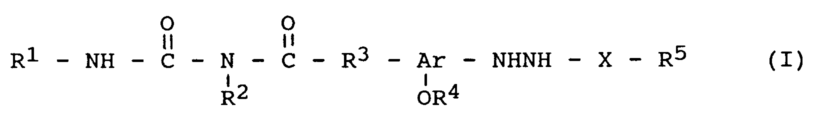 où
X est SO₂ ou CO,
R¹ et R² (qui peuvent être les mêmes ou différents) représentent chacun un groupe aliphatique ayant au moins 2 atomes de carbone, un groupe cycloaliphatique, un groupe aromatique substitué ou non ou un groupe hétérocyclique substitué ou non;
R³ représente -(CH₂)ₙ- où n est un entier de 1 à 4, de préférence 1 ou 2,
R⁴ représente un groupe aliphatique substitué ou non ou un groupe aromatique substitué ou non,
R⁵ représente l'hydrogène ou un groupe aliphatique, aromatique ou hétérocyclique qui peuvent éventuellement être substitués, avec la condition que lorsque X est SO₂, R⁵ ne soit pas un hydrogène,
Ar représente un groupe aromatique qui peut contenir des groupes alkyle ou alkoxy en plus de R³ et OR⁴.

7. Produit photographique selon la revendication 6 qui est un produit négatif à contraste élevé.

8. Procédé pour former une image photographique à contraste élevé consistant à exposer conformément à l'image un produit photographique selon les revendications 6 ou 7 et à le développer à un pH compris entre 10,5 et 12,0.
